# EUROPÄISCHE PATENTSCHRIFT

(11) **EP 0 433 816 B1**
(45) Veröffentlichungstag und Bekanntmachung des Hinweises auf die Patenterteilung: **20.04.1994**
(21) Anmeldenummer: 90123624.0
(22) Anmeldetag: 08.12.1990
(51) Int. Cl.: C07C 303/00, C07C 309/01, C07C 309/04

(54) **Verfahren und Vorrichtung zur Herstellung einer Lösung eines Buntmetallsulfonats**
Process and device for the preparation of a solution of a non-ferrous metal sulphonate
Procédé et dispositif pour la préparation d'une solution d'un sulfonate de métal lourd non-ferreux

(30) Priorität: 18.12.1989 DE 3941674
(43) Veröffentlichungstag der Anmeldung: 26.06.1991
(73) Patentinhaber: RIEDEL-DE HAEN AKTIENGESELLSCHAFT, D-30926 Seelze (DE)
(72) Erfinder: Remmers, Graalf, Dr., W-3000 Hannover 1 (DE); Lieker, Horst, W-3000 Hannover 1 (DE)
(74) Vertreter: Muley, Ralf, Dr.

(56) Entgegenhaltungen:
- GB-A- 1 472 735
- CHEMICAL ABSTRACTS, Band 81, Nr. 19, 11. November 1974, Columbus, Ohio,USAPAUL, RAM C. et al. "Chemistry of substituted sulfuric acids. VIII. Methan-sulfonates of tin(II), tin(IV), and zinc(II)" Seite 682, Zusammenfassung Nr.130 234n

## Beschreibung

Die vorliegende Erfindung betrifft ein Verfahren und eine Vorrichtung zur Herstellung einer wäßrigen Lösung eines Buntmetallsulfonats durch Umsetzung eines Buntmetalls mit einer Sulfonsäure.

Wäßrige Lösungen von bestimmten Buntmetallsulfonaten werden z.B. zur elektrolytischen oder stromlosen Abscheidung von Buntmetallen oder Buntmetallegierungen, zum elektrolytischen Färben von Aluminium und Aluminiumlegierungen und in elektrischen Batterien verwendet.

So wird z.B. beim elektrolytischen Färben von Aluminium und Aluminiumlegierungen nach der USP 4 128 460 (zitiert nach C.A. 92 (1980) 49412z) eine Lösung von Zinnmethansulfonat und Zinnpropansulfonat in Methansulfonsäure verwendet.

Zur gemeinsamen elektrolytischen Abscheidung von Zinn und Blei wird nach Jpn. Kokai Tokkyo Koho 59.211.562 (zitiert nach C.A. 102 (1985) 159,070u) eine saure Lösung von Zinnmethansulfonat und Bleimethansulfonat verwendet.

Aus Lösungen, die u.a. Zinnmethansulfonat bzw. das Zinnsalz einer Alkansulfonsäure, Hydroxyalkansulfonsäure, Benzolsulfonsäure oder Phenolsulfonsäure enthalten, kann Zinn stromlos abgeschieden werden, vgl. brit. Patentanmeldung 2 025 782 (zitiert nach C.A. 93, (1980) 52386v) bzw. Jpn. Kokai Tokkyo Koho 58.185759 (zitiert nach C.A. 100, (1984) 160.720w).

Wäßrige Bleimethansulfonat enthaltende Lösungen werden auch als Elektrolytlösungen in Batterien verwendet, vgl. z.B. südafrikanische Patentanmeldung 80,00051 (zitiert nach C.A. 95, (1981) 65268z).

Die Herstellung von Zinn(II)methansulfonat kann durch Umsetzung von Zinn(II)chlorid mit Methansulfonsäure erfolgen, vgl. C.A. 81, (1974) 130234n). Dieses Verfahren ist jedoch mehr für die präparative Darstellung im Labor geeignet. Die Herstellung von Zinn(II)methansulfonat nach diesem Verfahren bzw. von Buntmetallsulfonaten nach einem analogen Verfahren im industriellen Maßstabe ist zu umständlich und teuer. Die Herstellung von Buntmetallsulfonaten erscheint durch anodisches Lösen des Buntmetalls in einer mit der Sulfonsäure gefüllten Elektrolysezelle möglich. Auch sollten sich die gewünschten Salze durch Umsetzung der Sulfonsäuren mit den entsprechenden Buntmetalloxiden bzw. -hydroxiden darstellen lassen. So erscheint es möglich, Zinnmethansulfonat durch Umsetzen von Zinnoxid bzw. -hydroxid in Methansulfonsäure herzustellen. Auch diese Verfahren sind jedoch zu teuer, zum Teil liefern sie die gewünschten Salze lediglich mit Verunreinigungen verfrachtet und sind damit unwirtschaftlich.

Bei Versuchen, auf direktem Wege handelsüblich konzentrierte Zinn(II)methansulfonatlösungen durch Umsetzung von Zinnmetall mit Methansulfonsäure herzustellen, wurde festgestellt, daß unkontrollierte Nebenreaktionen eintreten. Diese führen zu einem extrem stark riechenden Reaktionsprodukt, das außerdem bräunlich gefärbte, irreversibel trübe Anteile enthält. Auch durch eine Filtration dieses Reaktionsprodukts kann kein akzeptables Endprodukt erhalten werden.

Aufgabe der vorliegenden Erfindung ist es, ein Verfahren und eine Vorrichtung zur einfachen und problemlosen Herstellung von wäßrigen Lösungen von Zinn(II)methansulfonat und anderen Zinnsulfonaten bzw. Buntmetallsulfonaten anzugeben, das ausgehend von geeigneten Sulfonsäuren, insbesondere von Methansulfonsäure, und geeigneten Buntmetallen, insbesondere von Zinn, die Buntmetallsulfonatlösungen in solcher Konzentration und Reinheit liefert, daß sie auf galvanotechnischen oder elektrochemischen Gebieten, z.B. zur Abscheidung der Buntmetalle oder als Elektrolyte in Batterien, geeignet sind.

Die Erfindung betrifft ein Verfahren zur Herstellung einer Lösung eines Buntmetallsulfonats durch Umsetzung eines Buntmetalls mit einer Sulfonsäure. Das erfindungsgemäße Verfahren ist dadurch gekennzeichnet, daß das Buntmetall mit einer Sulfonsäure bei einer Temperatur von 20 bis 120°C unter Einführen von Sauerstoff oder Sauerstoff enthaltenden Gasen in Kontakt gebracht wird. Vorzugsweise wird bei dem erfindungsgemäßen Verfahren ein Sauerstoff enthaltendes Gas und als solches vorzugsweise Luft verwendet.

Beispiele für geeignete Buntmetalle sind Kupfer, Nickel, Zink, Blei und Zinn, von denen Kupfer bevorzugt und das Zinn ganz besonders bevorzugt sind. Die vorliegende Erfindung eignet sich somit besonders zur Herstellung von wäßrigen Kupfer(II)sulfonat-Lösungen, vorzugsweise zur Herstellung von wäßrigen Zinn(II)sulfonat-Lösungen, vor allem wäßrigen Lösungen von Zinn(II)methansulfonat.

Als geeignete Sulfonsäuren kommen insbesondere Sulfonsäuren der folgenden allgemeinen Formel I in Betracht:

RSO₃H (I)

worin R einen Alkylrest mit 1 bis 12 C-Atomen, einen Hydroxyalkylrest mit 1 bis 10 C-Atomen, Phenyl oder Hydroxyphenyl bedeutet. Die für R stehenden Alkylreste und Hydroxyalkylreste können geradkettig oder verzweigt sein. Vorzugsweise sind sie geradkettig. Falls R für einen Hydroxyalkylrest steht, so hat dieser besonders bevorzugt die Formel: 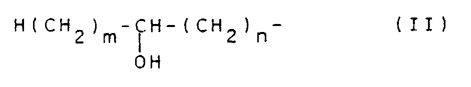
worin m die Zahl 0, 1 oder 2 und n eine ganze Zahl von 1 bis 8 bedeuten. Von den Sulfonsäuren der Formel I sind Alkansulfonsäuren mit 1 bis 12 C-Atomen, insbesondere geradkettige Alkansulfonsäuren mit 1 bis 6 C-Atomen, vorzugsweise Methan-, Ethan- und n-Propansulfonsäure besonders bevorzugt. Ganz besonders bevorzugt ist Methansulfonsäure.

Bei dem erfindungsgemäßen Verfahren wird, insbesondere bei der Herstellung Zinn(II)methansulfonat-Lösung, die Sulfonsäure vorzugsweise in einer wäßrigen Lösung eingesetzt, die mindestens 50 Gew.% Sulfonsäure, z.B. 50 bis 80 Gew.% Sulfonsäure enthält. Die Konzentration der zu Beginn eingesetzten wäßrigen Sulfonsäure beträgt zweckmäßigerweise 60 bis 70 Gew.%, vorzugsweise 60 bis 65 Gew.%, und insbesondere bei der Herstellung von Zinn(II)methansulfonat-Lösung ganz besonders bevorzugt 63,5 Gew.%.

Um eine möglichst hohe Umsetzungsgeschwindigkeit zu erreichen, wird das Buntmetall zweckmäßigerweise in Teilen mit großer Oberfläche eingesetzt, also z.B. als Schüttung von Schrot, Granalien oder Spänen. Zweckmäßigerweise wird verbrauchtes Buntmetall während der Reaktion wieder ersetzt. Die Umsetzung erfolgt vorzugsweise so, daß das Buntmetall zu Beginn der Reaktion vollständig von der wäßrigen Sulfonsäure bedeckt ist. Der Sauerstoff bzw. das Sauerstoff enthaltende Gas, vorzugsweise die Luft, wird vorzugsweise am Boden des Reaktionsbehälters in die Sulfonsäure eingeführt, z.B. eingepreßt, vorzugsweise eingeperlt. Durch das Inkontaktbringen der Sulfonsäure mit dem Buntmetall in Anwesenheit von Sauerstoff oder Sauerstoff enthaltendem Gas, insbesondere von Luft, beginnt die Bildung des Buntmetallsulfonats. Dadurch nimmt in der Reaktionsflüssigkeit der Gehalt an freier Sulfonsäure ab und und der Gehalt an Buntmetallsulfonat zu. Die Löslichkeit des Buntmetallsulfonats in der wäßrigen Sulfonsäure ist zu berücksichtigen, d.h. durch geeignete Wahl der Konzentration und der Temperatur bzw. Zugabe von Wasser ist ein Auskristallisieren des Buntmetallsulfonats in der Reaktionsflüssigkeit zu vermeiden.

Das Einführen bzw. Einpressen von Sauerstoff oder sauerstoffhaltigen Gasen, insbesondere von Luft, in die Reaktionsflüssigkeit kann in verschiedener Weise, z.B. über Lochplatten oder Düsen, Düsenstöcke etc., erfolgen. Normalerweise werden pro kg vorhandenem Buntmetall pro Stunde ca. 0,5 bis 5 l, vorzugsweise 1,5 bis 3 l, Sauerstoff oder ca. 2 bis 30 l, vorzugsweise 10 bis 15 l, Luft in die Reaktionsflüssigkeit eingeführt. Das Gewichtsverhältnis Buntmetall : wäßrige Sulfonsäure kann beispielsweise 1 : (2 bis 3) betragen.

Es ist zweckmäßig, die Reaktionsflüssigkeit während der Reaktion zu durchmischen. Diese Durchmischung der Reaktionsflüssigkeit erfolgt zweckmäßigerweise durch den in die Reaktionsflüssigkeit eingepreßten Sauerstoff oder durch das in die Reaktionsflüssigkeit eingepreßte sauerstoffhaltige Gas, insbesondere die Luft. Vorzugsweise erfolgt diese Durchmischung nach dem Mammutpumpenprinzip.

Es ist besonders zweckmäßig, die Reaktionsflüssigkeit durch eine Schüttung des Buntmetalls, insbesondere nach dem Mammutpumpenprinzip, umzuwälzen. Insbesondere wird ein Querschnittsverhältnis der Buntmetallschüttung zu dem restlichen Querschnitt, d.h. dem von Buntmetall freiem Querschnitt des Reaktors von 1 : (2 bis 6), vorzugsweise 1 : (3 bis 5) und insbesondere bei der Herstellung von Zinn(II)methansulfonat-Lösung ganz besonders bevorzugt von 1 : 4 eingehalten. Dieses Verhältnis wird entsprechend der Reaktionsfähigkeit des Buntmetalls gewählt. Die Reaktorhöhe bzw. die Höhe der Metallschüttung ergibt sich aus dem geforderten Nutzvolumen.

Die Reaktion wird vorzugsweise bei einer Temperatur von 70 bis 95°C, vorzugsweise 85 bis 90°C, durchgeführt. Höhere Temperaturen als 120°C können zur Zersetzung führen, andererseits geht bei niedrigen Temperaturen die Reaktionsgeschwindigkeit erheblich zurück.

Zweckmäßigerweise wird während der gesamten Reaktionszeit der Flüssigkeitsstand im Reaktor konstant gehalten, was durch Zufuhr von Wasser, vorzugsweise von Reinstwasser, bewirkt wird. Das zur Erhaltung des Flüssigkeitsniveaus zugeführte Wasser ist zweckmäßigerweise in seiner Temperatur der Reaktionstemperatur angepaßt. Es wird zweckmäßigerweise so zugeführt, daß in der Reaktionsflüssigkeit Hydrolysevorgänge vermieden werden. Zur Vermeidung von derartigen Hydrolysevorgängen ist bei der Zufuhr des Wassers eine möglichst schnelle und gute Durchmischung zu gewährleisten. Vorzugsweise wird das Wasser an der Oberfläche oder auf die Oberfläche der im Reaktor sich befindenden Reaktionsflüssigkeit zugeführt.

Das erfindungsgemäße Verfahren hat sich insbesondere zur Herstellung von Zinn(II)methansulfonat aus Zinn und Methansulfonsäure bewährt. Die angegebenen bevorzugten und besonders bevorzugten Maßnahmen und Daten sind besonders für die Umsetzung von Zinn und Methansulfonsäure geeignet.

Die Umsetzung dauert je nach den Ausgangskomponenten und der zur Anwendung kommenden Reaktionstemperatur mehrere Stunden bis mehrere Tage. Die nach der Beendigung der Umsetzung vorliegende Reaktionsflüssigkeit stellt eine wäßrige Buntmetallsulfonatlösung dar, die noch geringe Mengen an freier Sulfonsäure enthalten kann, die jedoch bei der Anwendung auf galvanotechnischen oder elektrochemischen Arbeitsgebieten nicht stören. Um beim Abkühlen ein Auskristallisieren des Buntmetallsulfonats zu vermeiden und um eine lagerstabile Lösung zu erhalten, wird die erhaltene Reaktionsflüssigkeit mit Wasser, z.B. auf Anwendungskonzentration, verdünnt. Z.B. beim Zinn(II)methansulfonat ist eine Lösung mit einem Zinngehalt von 22 Gew.% lagerstabil. Der Verdünnungsvorgang mit Wasser muß so erfolgen, daß keine Hydrolyse stattfindet. Dies kann dadurch bewirkt werden, daß die nach der Beendigung der Umsetzung vorliegende Reaktionsflüssigkeit möglichst rasch mit der benötigten Menge Wasser durchmischt wird, wobei das Verdünnungswasser zweckmäßigerweise über eine geeignete Dosiervorrichtung, z.B. eine Mischdüse, zudosiert wird.

Falls die erhaltene Buntmetallsulfonatlösung unerwünschte Anteile des Buntmetallsulfonats in höherer Oxidationsstufe enthält, dann kann die erhaltene Buntmetallsulfonatlösung zur Reduzierung des Buntmetallgehaltes der höheren Oxidationsstufe mit einer Schüttung des Buntmetalles in Kontakt gebracht werden, was zweckmäßigerweise so erfolgt, daß die erhaltene Reaktionslösung durch diese Buntmetallschüttung mechanisch, in Abwesenheit von Sauerstoff oder Luft, umgewälzt wird.

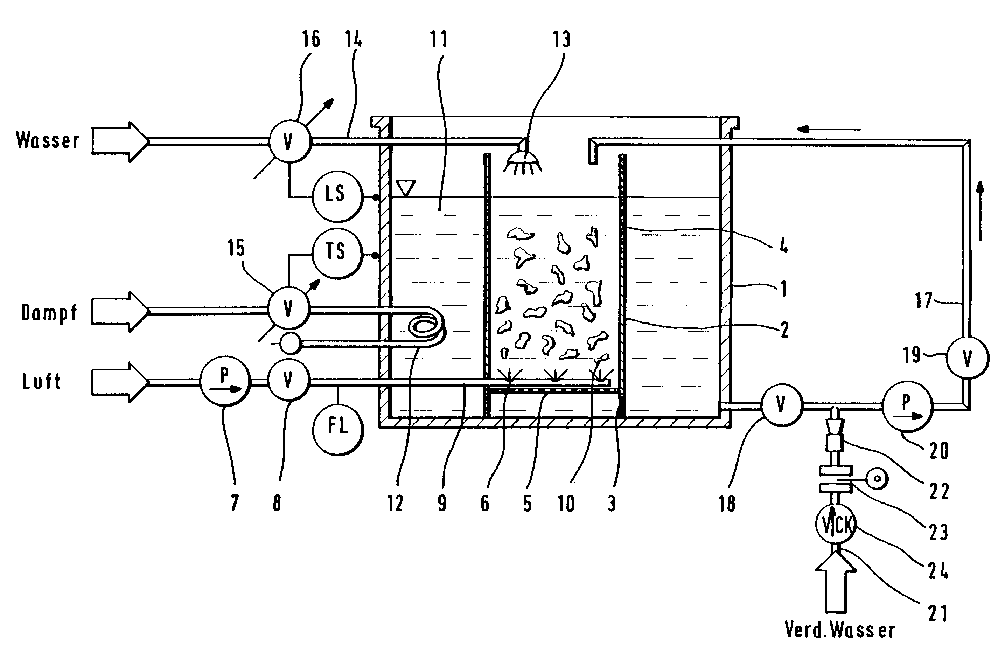

Anhand der beiliegenden Figur wird die Erfindung weiter erläutert. Die beiliegende Figur zeigt eine geeignete Vorrichtung zur Durchführung des erfindungsgemäßen Verfahrens in beispielsweiser und schematischer Darstellung.

Die Vorrichtung besteht aus dem eigentlichen Reaktionsbehälter 1 und dem Einsatz 2. Sowohl der Reaktionsbehälter 1 als auch der Einsatz 2 besitzen zweckmäßigerweise kreisförmige Querschnitte. Der Mantel des Einsatzes 2 besitzt an seinem unteren Ende Öffnungen 3 für den Durchtritt von Flüssigkeit. Am oberen Ende besitzt der Mantel des Einsatzes 2 in der Höhe des Flüssigkeitsspiegels Öffnungen 4 für den Durchtritt von Flüssigkeit. Im Inneren des Einsatzes 2 ist ein Boden 5 angeordnet, der ebenfalls Öffnungen für den Durchtritt von Flüssigkeit aufweist.

Im Inneren des Einsatzes 2 ist, beispielsweise über dem Boden 5, eine Vorrichtung 6, beispielsweise ein Düsenstock zum Einblasen von Sauerstoff, einem sauerstoffhaltigem Gas, insbesondere von Luft, angeordnet. Die Luft wird von dem Kompressor 7 über das Ventil 8 und die Leitung 9 zugeführt. Der Einsatz 2 wird mit einer Schüttung 10, d.h. z.B. mit Granalien, Spänen und dergleichen des Buntmetalls bis zur Höhe des Beginns der Öffnungen 4 gefüllt. Anschließend wird der Reaktor 1 vorzugsweise mit mindestens 50 gew.%iger wäßriger Sulfonsäure bis zu der eingezeichneten Höhe gefüllt. In die Flüssigkeit 11 taucht eine Heizvorrichtung 12, durch die Wärme zur Aufrechterhaltung der erforderlichen Reaktionstemperatur zugeführt wird. Zweckmäßigerweise wird die Einhaltung der Reaktionstemperatur automatisch geregelt. Bei der dargestellten Vorrichtung wird die Temperatur durch Zufuhr von Dampf in die Heizvorrichtung 12 eingehalten.

Über dem Flüssigkeitsspiegel, zweckmäßigerweise im Inneren Einsatzes 2 befindet sich eine Vorrichtung 13 für die Zufuhr von Wasser, insbesondere von Reinstwasser. Die Vorrichtung 13 kann beispielsweise als Brause oder dergl. ausgestaltet sein. Über die Vorrichtung 13 und die zugehörige Leitung 14 wird Wasser zur Konstanthaltung des Flüssigkeitsspiegels in der Vorrichtung 1 zugeführt. Zweckmäßigerweise wird die Wasserzufuhr in Abhängigkeit von dem Flüssigkeitsstand automatisch geregelt.

Nach Einbringung des Buntmetalls in das Innere des Einsatzes 2 und das Einfüllen der wäßrigen Sulfonsäure wird die Sulfonsäure auf die erforderliche Reaktionstemperatur aufgeheizt und über den Düsenstock 6 Luft eingeblasen. Dadurch wird die im Einsatz 2 vorhandene Flüssigkeit nach oben durch die Buntmetallschüttung 10 bewegt. Die Flüssigkeit tritt anschließend durch die Öffnungen 4 in den äußeren Ringraum des Reaktors 1 über, wo sie von oben nach unten strömt und über die Öffnungen 3 und den flüssigkeitsdurchlässigen Boden 5 wieder in den Einsatz 2 und in die Buntmetallschüttung 10 zurückströmt. Im Reaktor 1 findet daher eine durch das Mammutpumpenprinzip bewirkte Umlaufströmung statt. Verbrauchtes Buntmetall wird während der Reaktion ersetzt, und der Flüssigkeitstand im Reaktor wird während der gesamten Reaktionszeit durch Zufuhr von Wasser, insbesondere von Reinstwasser, über die Vorrichtung 13 konstant gehalten.

In der Figur ist das Regelventil, das die Zufuhr von Dampf in die Heizvorrichtung 12 regelt, mit 15 und das Regelventil für die Zuführung des Wassers mit 16 bezeichnet.

Die Vorrichtung besitzt noch eine Bypassleitung 17 mit den Ventilen 18 und 19 und der Pumpe 20. Über die Bypassleitung 17 kann Flüssigkeit nahe des Behälterbodens entnommen und auf den Flüssigkeitsspiegel im Reaktionseinsatz 2 zurückgeführt werden. In die Bypassleitung 17 mündet die Leitung 21, in welche die Düse 22 und die Kaliberscheibe 23 eingebaut ist. Über die Leitung 21 und die Kaliberscheibe 23, Düse 22, die als Dosiereinrichtung verwendet wird, wird nach Beendigung der eigentlichen Reaktion Verdünnungswasser zugeführt. Um einen Rückstau von Salzkonzentrat in die Wasservorlage zu verhindern, ist die Wasserzufuhr mit einem Rückschlagventil 24 gesichert.

In die Bypassleitung 17 kann eine Buntmetallschüttung eingeschaltet werden, um nach Beendigung der Reaktion durch Umpumpen der Reaktionslösung über den Bypass und die in den Bypass 17 eingeschaltete Buntmetallschüttung den Gehalt höherer Oxidationsstufen zu reduzieren.

Sämtliche mit der wäßrigen Sulfonsäure bzw. der Reaktionsflüssigkeit in Berührung kommenden Teile der Vorrichtung müssen aus Materialien hergestellt sein oder mit Materialien beschichtet sein, die gegenüber der Sulfonsäure und der entstehenden Salzlösung inert sind. Derartige inerte Materialien sind z.B. Glas, porzellan, Keramik, Steingut, ferner z.B. Kunststoffe, wie z.B. Polyethylen, Polypropylen, Polyvinylchlorid und Fluorkunststoffe. Als Fluorkunststoffe kommen beispielsweise fluorhaltige polymerisate, wie z.B. Polytetrafluorethylen (PTFE), Polytrifluorchlorethylen (PCTFE), Polyvinylfluorid (PVF), Polyvinylidenfluorid (PVDF) und Mischpolimerisate, wie z.B. Tetrafluorethylen-Perfluorpropylen-Mischpolymerisate (FEP), Tetrafluorethylen-Perfluoralkylvinylether-Mischpolymerisate (PFA/TFA), Trifluorchlorethylen-Ethylen-Mischpolymerisate (ETFE/CTFE) in Betracht. Besonders geeignet als Fluorkunststoffe sind z.B. polytetrafluorethylen (PTFE), polyvinylidendifluorid (PVDF) und Tetrafluorethylen-Perfluoralkylvinyl-Mischpolymerisate (PFA/TFA).

Zweckmäßigerweise wird die Innenseite des Reaktors und die mit Flüssigkeit in Berührung kommenden Teile des Einsatzes möglichst glatt ausgebildet.

Das Verhältnis des Querschnitts des Einsatzes 2 zu dem Querschnitt der nicht von dem Einsatz 2 bedeckten Reaktorfläche, die bei zylindrischem Reaktor 1 und zylindrischem Einsatz 2 durch den Ringraum zwischen der Wand des Reaktors 1 und dem Einsatz 2 gegeben ist, beträgt in vielen Fällen 1 : (2 bis 6), vorzugsweise 1 : (3 bis 5) und ganz besonders 1 : 4. Das Verhältnis von 1 : 4 hat sich besonders bei der Umsetzung von Zinn mit Methansulfonsäure als vorteilhaft bewährt.

### Beispiel

In der in der Figur dargestellten Vorrichtung wird aus Zinn und Methansulfonsäure eine wäßrige Zinn(II)methansulfonat-Lösung hergestellt. In den Einsatz 2 wird eine Zinnschüttung von 1000 kg granuliertem Zinn eingebracht und anschließend der Reaktor 1 mit 2605 kg wäßriger Methansulfonsäure mit einem Gehalt von 63,5 Gew.% gefüllt. Über den Düsenstock 6 werden 10 bis 15 cm³/h Luft eingeleitet, und die Reaktion bei einer Temperatur von 85 bis 90°C 15 Tage lang durchgeführt. Die Reaktion wird beendet, wenn eine wäßrige Lösung mit ca. 25 Gew.% Zinn und ca. 7 bis 9 Gew.% freier Methansulfonsäure vorliegt. Um ein Auskristallisieren des Zinn(II)methansulfonats bei Raumtemperatur zu vermeiden, wird der flüssige Reaktorinhalt mit Wasser verdünnt, bis eine lagerstabile Lösung mit einem Zinngehalt von 22 Gew.% erhalten wird. Die Verdünnung erfolgt durch Umwälzung der Reaktionsflüssigkeit über den Bypass 17, wobei über die kalibrierte Düse 22 Wasser im Volumenverhältnis 1 : 30 zudosiert wird.

Die zunächst erhaltene Zinn(II)methansulfonatlösung kann Zinn(IV)-Anteile in der Größenordnung von ca. 0,8 Gew.% enthalten. Da für galvanotechnische Anwendungen ein möglichst geringer Zinn(IV)-Gehalt wünschenswert ist, wird die erhaltene Lösung über den Bypass 17 über eine reduzierende Zinngranalienschüttung umgewälzt, wobei zweckmäßigerweise ebenfalls eine Temperatur von über 80°C, insbesondere 85 bis 90°C, eingehalten wird. Es wird so lange über die reduzierende Zinngranalienschüttung umgewälzt, bis der Zinn(IV)-Gehalt auf den gewünschten Wert gefallen ist. In vielen Fällen sind Zinn(IV)-Gehalte von unter 0,1 Gew.% ausreichend.

Auf die beschriebene Weise wird eine wäßrige Zinn(II)methansulfonatlösung mit ca. 22 Gew.% Zinn und einem Zinn(IV)-Gehalt von unter 0,1 Gew.% erhalten, die allen galvanotechnischen Anforderungen genügt.

## Patentansprüche

1. Verfahren zur Herstellung einer Lösung eines Buntmetallsulfonats durch Umsetzung eines Buntmetalls mit einer Sulfonsäure, dadurch gekennzeichnet, daß das Buntmetall mit einer Sulfonsäure bei einer Temperatur von 20 bis 120°C unter Einführen von Sauerstoff oder Sauerstoff enthaltenden Gasen in Kontakt gebracht wird.

2. Verfahren nach Anspruch 1, dadurch gekennzeichnet, daß die Reaktionsflüssigkeit durch den eingeführten Sauerstoff oder das eingeführte Sauerstoff enthaltende Gas durchmischt und insbesondere nach dem Mammutpumpenprinzip umgewälzt wird.

3. Verfahren nach Anspruch 1 und/oder 2, dadurch gekennzeichnet, daß die Reaktionsflüssigkeit durch eine Schüttung des Buntmetalls umgewälzt wird.

4. Verfahren nach einem oder mehreren der Ansprüche 1 bis 3, dadurch gekennzeichnet, daß als Sauerstoff enthaltendes Gas Luft verwendet wird.

5. Verfahren nach einem oder mehreren der Ansprüche 1 bis 4, dadurch gekennzeichnet, daß als Buntmetall Zinn, vorzugsweise in Form von Granalien oder Spänen, eingesetzt wird.

6. Verfahren nach einem oder mehreren der Ansprüche 1 bis 5, dadurch gekennzeichnet, daß eine wäßrige Sulfonsäure eingesetzt wird, die mindestens 50 Gew.%, vorzugsweise 60 bis 70 Gew.%, vorzugsweise 60 bis 65 Gew.% und ganz besonders bevorzugt 63,5 Gew.%, Sulfonsäure enthält.

7. Verfahren nach einem oder mehreren der Ansprüche 1 bis 6, dadurch gekennzeichnet, daß die Reaktion bei einer Temperatur von 70 bis 95°C, vorzugsweise 85 bis 90°C, durchgeführt wird.

8. Verfahren nach einem oder mehreren der Ansprüche 1 bis 7, dadurch gekennzeichnet, daß die Reaktion in einem Reaktor durchgeführt wird, in dem das Verhältnis des Querschnitts Buntmetallschüttung zu dem restlichen Querschnitt des Reaktors 1 : (2 bis 6), vorzugsweise 1 : (3 bis 5) und ganz besonders bevorzugt 1 : 4 beträgt.

9. Verfahren nach einem oder mehreren der Ansprüche 1 bis 8, dadurch gekennzeichnet, daß als Sulfonsäure eine Sulfonsäure der Formel I
RSO₃H (I)
eingesetzt wird, worin R einen Alkylrest mit 1 bis 12 C-Atomen, einen Hydroxyalkylrest mit 1 bis 10 C-Atomen, Phenyl oder Hydroxyphenyl bedeutet.

10. Verfahren nach einem oder mehreren der Ansprüche 1 bis 9, dadurch gekennzeichnet, daß als Sulfonsäure Methansulfonsäure eingesetzt wird.

11. Verfahren nach einem oder mehreren der Ansprüche 1 bis 10, dadurch gekennzeichnet, daß die Höhe des Flüssigkeitsspiegels während der Umsetzung durch Zufuhr von Wasser konstant gehalten wird.

12. Verfahren nach einem oder mehreren der Ansprüche 1 bis 11, dadurch gekennzeichnet, daß die nach Beendigung der Umsetzung vorliegende Flüssigkeit mit Wasser auf Anwendungskonzentration verdünnt und/oder ohne Zufuhr von Sauerstoff oder Sauerstoff enthaltendem Gas durch eine Schüttung des Buntmetalls umgewälzt wird.

13. Vorrichtung zur Durchführung des in einem oder mehreren der Ansprüche 1 bis 12 angegebenen Verfahrens, gekennzeichnet durch einen Reaktor (1), in dem ein Einsatz (2) für die Aufnahme der Schüttung (10) des Buntmetalls angeordnet ist, und durch eine Vorrichtung (6) für das Einpressen von Sauerstoff oder einem Sauerstoff enthaltenden Gas, durch eine Heizvorrichtung (12), eine Vorrichtung (13) zum Zuführen von Wasser und einem Bypass (17).

14. Vorrichtung nach Anspruch 13, dadurch gekennzeichnet, daß der Reaktor (1) und der Einsatz (2) kreisförmige Querschnitte besitzen und der Einsatz (2) zentrisch im Reaktor (1) angeordnet ist.

15. Vorrichtung nach Anspruch 13 und/oder 14, dadurch gekennzeichnet, daß das Verhältnis des Querschnitts des Einsatzes (2) zu der restlichen Querschnittsfläche des Reaktors 1 : (2 bis 6), vorzugsweise 1 : (3 bis 5) und ganz besonders bevorzugt 1 : 4 beträgt.

## Claims

1. Process for preparing a solution of non-ferrous metal sulphonate by reacting a non-ferrous metal with a sulphonic acid, characterised in that the non-ferrous metal is brought into contact with a sulphonic acid at a temperature of from 20 to 120°C while introducing oxygen or oxygen-containing gases.

2. Process according to Claim 1, characterised in that the reaction liquid is mixed by the introduced oxygen or the introduced oxygen-containing gas and is circulated, in particular, by the airlift principle.

3. Process according to Claim 1 and/or 2, characterised in that the reaction liquid is circulated through a bed of the non-ferrous metal.

4. Process according to one or more of Claims 1 to 3, characterised in that the oxygen-containing gas used is air.

5. Process according to one or more of Claims 1 to 4, characterised in that the non-ferrous metal employed is tin, preferably in the form of granules or turnings.

6. Process according to one or more of Claims 1 to 5, characterised in that an aqueous sulphonic acid is employed which contains at least 50 % by weight, preferably 60 to 70 % by weight, preferably 60 to 65 % by weight, and very particularly preferably 63,5 % by weight, of sulphonic acid.

7. Process according to one or more of Claims 1 to 6, characterised in that the reaction is carried out at a temperature of from 70 to 95°C, preferably 85 to 90°C.

8. Process according to one or more of Claims 1 to 7, characterised in that the reaction is carried out in a reactor in which the ratio between the cross-section of the non-ferrous metal bed and the remaining cross-section of the reactor is 1 : (2 to 6), preferably 1 : (3 to 5) and very particularly preferably 1 : 4.

9. Process according to one or more of Claims 1 to 8, characterised in that the sulphonic acid employed is a sulphonic acid of the formula I
RSO₃H (I)
in which R denotes an alkyl radical having 1 to 12 C atoms, a hydroxyalkyl radical having 1 to 10 C atoms, phenyl of hydroxyphenyl.

10. Process according to one or more of Claims 1 to 9, characterised in that the sulphonic acid amployed is methanesulphonic acid.

11. Process according to one or more of Claims 1 to 10, characterised in that the liquid level is kept constant during the reaction by adding water.

12. Process according to one or more of Claims 1 to 11, characterised in that the liquid present after completion of the reaction is diluted with water to the use concentration and/or circulated through a bed of the non-ferrous metal without supply of oxygen or an oxygen-containing gas.

13. Apparatus for carrying out the process indicated in one or more of Claims 1 to 12, characterised by a reactor (1) in which an insert (2) for accommodating the bed (10) of the non-ferrous metal is arranged, and by a device (6) for injecting oxygen or an oxygen-containing gas, by a heating device (12), a device (13) for supplying water, and a bypass (17).

14. Apparatus according to Claim 13, characterised in that the reactor (1) and the insert (2) have circular cross-sections, and the insert (2) is arranged centrally in the reactor (1).

15. Apparatus according to Claim 13 and/or 14, characterised in that the ratio between the cross-section of the insert (2) and the remaining cross-sectional area of the reactor is 1 : (2 to 6), preferably 1 : (3 to 5) and very particularly preferably 1 : 4.

## Revendications

1. Procédé de préparation d'une solution d'un sulfonate de métal lourd non-ferreux par réaction du métal avec un acide sulfonique, procédé caractérisé en ce que l'on fait réagir par mise en contact le métal avec un acide sulfonique à une température de 20 à 120°C, tout en y faisant arriver de l'oxygène ou un gaz contenant de l'oxygène.

2. Procédé selon la revendication 1, caractérisé en ce que l'on mélange le liquide de réaction par l'arrivée d'oxygène ou du gaz oxygéné, en particulier suivant la technique de la pompe mammouth.

3. Procédé selon la revendication 1 et/ou 2, caractérisé en ce que l'on fait circuler le liquide de réaction par des fragments du métal.

4. Procédé selon une ou plusieurs des revendications 1 à 3, caractérisé en ce que l'on utilise de l'air comme gaz oxygéné.

5. Procédé selon une ou plusieurs des revendications 1 à 4, caractérisé en ce que le métal lourd non ferreux est de l'étain, de préférence en grenaille, tournure ou copeaux.

6. Procédé selon une ou plusieurs des revendications 1 à 5, caractérisé en ce que l'on opère avec un acide sulfonique aqueux à 50% en poids au moins d'acide sulfonique, de préférence de 60 à 70%, mieux encore de 60 à 65%, et tout spécialement de 63,5% en poids.

7. Procédé selon une ou plusieurs des revendications 1 à 6, caractérisé en ce que l'on effectue la réaction à une température de 70 à 95°C, de préférence de 85 à 90°C.

8. Procédé selon une ou plusieurs des revendications 1 à 7, caractérisé en ce que l'on opère dans un réacteur dont le rapport de la section transversale du récipient de charge des morceaux de métal à la section restante du réacteur est de 1:(2 à 6), de préférence de 1:(3 à 5), et tout spécialement de 1:4.

9. Procédé selon une ou plusieurs des revendications 1 à 8, caractérisé en ce que l'acide sulfonique est un acide de formule I :
RSO₃H (I)
R désignant un alkyle ayant de 1 à 12 atomes de carbone, un hydroxyalkyle ayant de 1 à 10 atomes de carbone ou un groupe phényle ou hydroxyphényle.

10. Procédé selon une ou plusieurs des revendications 1 à 9, caractérisé en ce que l'acide sulfonique est de l'acide méthane-sulfonique.

11. Procédé selon une ou plusieurs des revendications 1 à 10, caractérisé en ce que au cours de la réaction on maintient le niveau de liquide constant par une arrivée d'eau.

12. Procédé selon une ou plusieurs des revendications 1 à 11, caractérisé en ce, quand la réaction est terminée, on dilue le liquide à l'eau à sa concentration d'emploi et/ou, sans faire arriver d'oxygène ou de gaz oxygéné, on le fait circuler par des fragments du métal lourd.

13. Appareil pour l'exécution du procédé décrit à une ou plusieurs des revendications 1 à 12, caractérisé en ce qu'il comprend un réacteur (1) dans lequel est placé un récipient de charge (2) des fragments du métal lourd (10), un dispositif (6) pour l'introduction sous pression d'oxygène ou d'un gaz contenant de l'oxygène, un dispositif de chauffage (12), un dispositif (13) d'arrivée d'eau et un by-pass (17).

14. Appareil selon la revendication 13, caractérisé en ce que le réacteur (1) et le récipient de charge (2) ont une section transversale circulaire et le récipient (2) est situé au centre du réacteur (1).

15. Appareil selon la revendication 13 et/ou 14, caractérisé en ce que le rapport de la section transversale du récipient de charge (2) à la surface de section transversale restante du réacteur (1) est de 1:(2 à 6), de préférence de 1:(3 à 5) et tout spécialement de 1:4.
